# FASCICULE DE BREVET EUROPEEN

(11) **EP 0 320 370 B1**
(45) Date de publication et mention de la délivrance du brevet: **10.11.1993**
(21) Numéro de dépôt: 88403089.1
(22) Date de dépôt: 06.12.1988
(51) Int. Cl.: C07D 403/14, C06B 25/04

(54) **5-Nitro-4,6-bis-((5-nitro-1H-1,2,4-triazol-3-yl)amino)pyrimidine, son procédé de préparation et matériau explosif le contenant**
4,6-Di-(3-amin-5-nitro-1,2,4-triazol)-5-nitropyrimidinderivat, Verfahren zu seiner Herstellung und dieses enthaltender Sprengstoff
4,6-Di-(3-amine-5-nitro-1,2,4-triazol)-5-nitropyrimidine derivative, process for its preparation and explosive material containing it

(30) Priorité: 08.12.1987 FR 8717059
(43) Date de publication de la demande: 14.06.1989
(73) Titulaire: COMMISSARIAT A L'ENERGIE ATOMIQUE, 75015 Paris Cédex 15 (FR)
(72) Inventeur: Laval, François, F-37260 Monts (FR); Wartenberg, Christian, F-37260 Monts (FR); Morignat, Marie-Louise, F-37100 Tours (FR)
(74) Mandataire: Mongrédien, André

(56) Documents cités:
- US-A- 2 987 520
- US-A- 3 483 211
- US-A- 3 923 804

## Description

La présente invention a pour objet un nouveau dérivé de pyrimidine, son procédé de fabrication et son utilisation comme explosif.

De façon plus précise, elle concerne un nouveau dérivé de pyrimidine utilisable comme explosif secondaire, pour l'équipement de missiles et d'armements modernes.

Pour ces applications, il est souvent intéressant d'utiliser des explosifs ayant une sensibilité au choc aussi faible que possible, mais une puissance élevée, c'est-à-dire une capacité de délivrer une énergie très élevée. Ces deux propriétés sont difficiles à trouver simultanément dans un même explosif. Ainsi, le triaminotrinitrobenzène (TATB) est très peu sensible au choc, mais il manque de puissance, alors que la cyclotétraméthylène tétranitramine (octogène) très puissante est, par contre, plus sensible au choc et aux agressions.

C'est pour cette raison que des recherches sont actuellement effectuées afin de mettre au point de nouveaux explosifs dont la sensibilité au choc soit voisine de celle du TATB, tout en étant capables de délivrer une énergie plus élevée que ce dernier, se rapprochant de celle de l'octogène.

La présente invention a précisément pour objet un nouveau dérivé de pyrimidine présentant ces propriétés.

Le document US-A- 3 923 804 illustre des matériaux explosifs comportant un noyau pyrimidine substitué par des groupes trinitrophényle, qui sont différents des dérivés de pyrimidine de l'invention.

Le nouveau dérivé de pyrimidine de l'invention est la 4,6-di-(3-amino-5-nitro-1,2,4-triazole)-5-nitropyrimidine obtenue par réaction du sel de sodium du 3-amino-5-nitro-1,2,4-triazole avec la 4,6-dichloro-5-nitropyrimidine, caractérisé par un point de fusion de 310°C (avec décomposition), une densité mesurée au picnomètre à gaz de 1,85 et une surface spécifique de 2,14m²/g.

Ce nouveau dérivé de pyrimidine présente un grand intérêt pour une utilisation comme explosif secondaire car ses propriétés détoniques sont intermédiaires entre celles du TATB et celles de l'octogène, en ce qui concerne la sensibilité au choc et la vitesse de détonation.

Ce nouveau dérivé de pyrimidine est préparé par un procédé consistant à faire réagir la 4,6-dichloro-5-nitropyrimidine de formule : 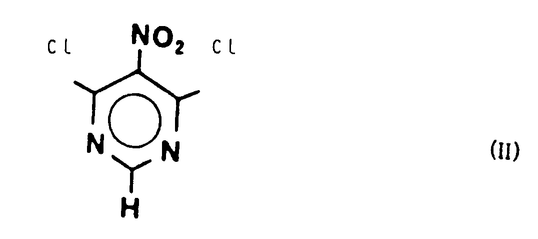
, avec le sel de sodium du 3-amino-5-nitro-1,2,4-triazole
Ce procédé est basé sur une réaction de substitution nucléophile entre le 3-amino-5-nitro-1,2,4-triazole et la 4,6-dichloro-5-nitropyrimidine de formule II qui sont des réactifs de départ disponibles sur le marché ou de synthèse aisée.

Le sel de sodium du 3-amino-5-nitro-1,2,4-triazole peut être obtenu en soumettant le 3-amino-1,2,4-triazole à une acétylation pour former le dérivé 3-acétamido-1,2,4-triazole, et en réalisant ensuite une nitration du 3-acétamido-1,2,4-triazole ainsi obtenu pour former le 3-amino-5-nitro-1,2,4-triazole que l'on fait réagir dans de l 'éthanol absolu avec du sodium.

Le 3-amino-1,2,4-triazole est un produit commercial utilisé dans les industries phytosanitaires et photographiques, et donc facilement disponible.

Avant la nitration effective, la fonction amine du 3-amino-1,2,4-triazole est protégée par acétylation : 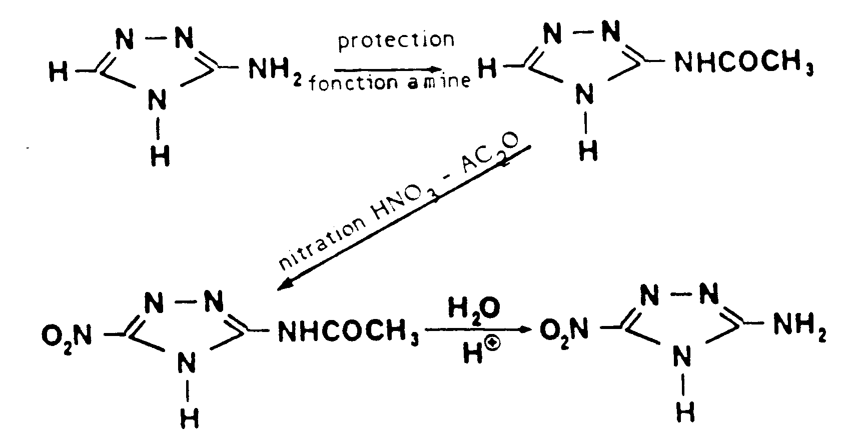
L'ensemble de cette synthèse est décrit par M.S. PEVZNER et Co. dans Khimiya Geterotsiklicheskikh Soedinenii, N^{o}8, pp.1132-35, 08/79.

Dans le procédé de l'invention on utilise la 4,6-dichloro-5-nitropyrimidine, en raison de sa bonne disponibilité commerciale et de la grande réactivité de ses deux atomes de chlore.

Pour réaliser la réaction, on synthétise tout d'abord le sel de sodium du 3-amino-5-nitro-1,2,4-triazole en plaçant le 3-amino-5-nitro-1,2,4-triazole dans une solution d'alcoolate alcalin, de préférence d'éthanolate de sodium. Le sel de sodium est obtenu après chauffage au reflux durant 30 min. La 4,6-dichloro-5-nitropyrimidine est alors ajoutée en solution alcoolique par exemple dans l'éthanol absolu.

La réaction est obtenue après reflux durant trois heures et le composé final est débarrassé du chlorure de sodium par lavages successifs dans l'eau sous ultrasons.

Le dérivé de pyrimidine de l'invention peut être utilisé comme matériau explosif. Dans ce cas, ce dérivé est généralement dispersé dans un liant thermoplastique ou thermodurcissable contenant éventuellement d'autres additifs habituellement utilisés dans de telles compositions (plastifiants, colorants, etc..).

D'autres caractéristiques et avantages de l'invention apparaîtront mieux à la lecture de l'exemple suivant donné, bien entendu, à titre illustratif.

Cet exemple illustre la préparation de la 4,6-di-(3-amino-5-nitro-1,2,4-triazole)-5-nitropyrimidine à partir de la 4,6-dichloro-5-nitropyrimidine du commerce et du 3-amino-5-nitro-1,2,4-triazole que l'on obtient à partir du 3-amino-1,2,4-triazole.

### 1. Préparation du 3-amino-5-nitro-1,2,4-triazole.

Pour cette préparation, on part de 3-amino-1,2,4-triazole que l'on soumet tout d'abord à une acétylation pour protéger la fonction amine et former le dérivé 3-acétamido-1,2,4-triazole, ce dérivé est ensuite soumis à une nitration.

### a) Préparation du 3-acétamido-1,2,4-triazole

A 3 litres d'anhydride acétique préchauffé vers 30-35°C, on ajoute progressivement 1000g d'aminotriazole. En fin d'introduction, on porte à ébullition pendant 10 min. Après retour à la température ambiante, on filtre et on lave avec 1 litre de toluène ; le précipité est ensuite séché puis hydrolysé avec 3 litres d'eau. L'ensemble est porté à 80°C durant une nuit.

On filtre alors le 3-acétamido-1,2,4-triazole obtenu, puis on le lave à l'eau et on le sèche.

Le rendement global se si tue vers 85-87%.

### b) Préparation du 3-amino-5-nitro-1,2,4-triazole

Dans un mélange de 224 ml d'anhydride acétique et 100 ml d'acide nitrique 100% (d=1,52) refroidi à -20°C, on introduit progressivement 60 g du 3-acétamido-1,2,4-triazole obtenu en a) et on maintient l'ensemble à cette température durant 30 min. puis on le réchauffe progressivement jusqu'à -5°C.

La solution est alors précipitée sur de la glace, filtrée pour éliminer les sous-produits et le filtrat est extrait à l'acétate d'éthyle. La phase organique est concentrée et le 3-amino-5-nitro-1,2,4-triazole précipite. Celui-ci est filtré puis lavé à l'éther éthylique. Le rendement global de la nitration se situe vers 30%.

### 2. Préparation de la 4,6-di-(3-amino-5-nitro-1,2,4-triazole)-5-nitropyrimidine.

A 100 ml d'éthanol absolu, on ajoute par petites fractions 0,92g de sodium, sous agitation, jusqu'à dissolution de l'ensemble. On introduit ensuite 5,16g de l'aminonitrotriazole obtenu précédemment et on porte à reflux durant une heure pour préparer le sel alcalin de formule (IV).

On introduit ensuite 3,88g de 4,6-dichloro-5-nitropyrimidine dissous dans environ 60 ml d'éthanol absolu et on porte à reflux durant 3 heures. On filtre le produit obtenu et on élimine le NaCl formé par épuisements successifs dans l'eau sous ultrasons.

Le rendement global est de 60%. L'analyse élémentaire du produit obtenu est la suivante :

| | C | H | N | O |
|---|---|---|---|---|
| Trouvée | 25,43 | 1,35 | 47,80 | 25,35 |
| Calculée | 25,34 | 1,33 | 48,02 | 25,31 |

Le produit obtenu se présente sous l'aspect d'un solide jaune clair cristallisé dont le point de fusion est à 310°C (décomposition).

Sa densité, mesurée au picnomètre à gaz, est ρ = 1,85. Il est soluble dans l'acide sulfurique, et dans de très faibles proportions, dans la N-méthylpyrrolidone et le diméthylsulfoxyde (DMSO). Il est insoluble dans les solvants chlorés, aromatiques, l'acétone, l'eau, les alcools et le diméthylformamide (DMF).

Sa surface spécifique est de 2,14 m²/g (mesurée par la méthode BET).

Il présente les propriétés suivantes :

### I. Propriétès spectroscopiques

### 1) Résonance magnétique Nucléaire (RMN)

La faible solubilité du produit n'a permis que d'effectuer un spectre proton ¹H à 60 MHz de l'échantillon en solution dans le diméthylsulfoxyde (DMSOD₆).

Celui-ci comporte deux pics :
- 1 pic à 9,12 ppm, et
- 1 pic à 8,70 ppm.

### 2) Spectre infrarouge

Le spectre infrarouge fait apparaître un ensemble de bandes difficilement attribuables indiquant la présence de cycles aromatiques nitrés. On observe également un large massif entre 2800 et 3600 cm⁻¹, domaine des νNH.

### II. Propriétés thermiques et détoniques

### 1) Balance en oxygène.

La balance en oxygène par rapport au CO₂ et H₂O est de -52,74g d'O₂ pour 100g de produit. A titre comparatif, la balance en oxygène du TATB est de -55,81g/100g et celle de l'octogène de -21,6 g/100g.

### 2) Température de déflagration.

Un échantillon d'environ 20 mg en conteneur d'acier inoxydable est trempé dans un bain dont la température est élevée à 5°C/min. Dans le cas présent, la température à laquelle le produit déflagre est de 310-311°C.

### 3) Temps d'induction thermique.

On introduit brusquement dans un bain à la température de mesure environ 10mg du produit dans un conteneur d'acier. On enregistre le temps au bout duquel on a décomposition. En considérant que la cinétique est d'ordre zéro, on peut calculer l'énergie d'activation.

Pour le 4,6-di-(3-amino-5-nitro-1,2,4-triazole)-5-nitropyrimidine, la déflagration se produit au bout de 5s pour une température de 356°C.

L'énergie d'activation de la réaction est de 44,05 kcal/mole.

### 4) Vitesse de détonation.

La vitesse de détonation calculée à partir de la formule brute, selon la méthode empirique de Rothstein et Petersen décrite dans Propellants and Explosives 4, 56-60 (1979) est, pour la densité de cristal, de 8600 m/s. A titre comparatif, selon cette même méthode, la vitesse de détonation calculée du TATB est de 7870 m/s et celle de l'octogène de 9050 m/s.

### 5) Sensibilité au choc.

La sensibilité au choc du produit a été déterminée à l'aide d'un mouton pendulaire de 5kg, un échantillon de 30 mg étant déposé sur papier de verre. La conduite de l'essai se fait selon la méthode de Bruceton.

Dans de telles conditions d'essai, la probabilité de 50% de réaction pyrotechnique a été obtenue pour la hauteur de 61,5 cm. A titre comparatif, la sensibilité au choc du TATB correspond à H₅₀>72 cm et celle de l'octogène à H₅₀=15 cm.

On constate ainsi que la 4,6-di-(3-amino-5-nitro-1,2,4-triazole)-5-nitropyrimidine a une sensibilité au choc faible tout en étant capable de délivrer une énergie élevée.

## Revendications

1. 4,6-di-(3-amino-5-nitro-1,2,4-triazole)-5-nitropyrimidine obtenue par réaction du sel de sodium du 3-amino-5-nitro-1,2,4-triazole avec la 4,6-dichloro-5-nitropyrimidine, caractérisée par un point de fusion de 310°C (avec décomposition), une densité mesurée au picnomètre à gaz de 1,85 et une surface spécifique de 2,14m²/g.

2. 4,6-di-(3-amino-5-nitro-1,2,4-triazole)-5-nitropyrimidine selon la revendication 1, caractérisée en ce que le sel de sodium du 3-amino-5-nitro-1,2,4-triazole est obtenu en soumettant le 3-amino-1,2,4-triazole à une acétylation pour former le dérivé 3-acétamido-1,2,4-triazole, et en réalisant ensuite une nitration du 3-acétamido-1,2,4-triazole ainsi obtenu pour former le 3-amino-5-nitro-1,2,4-triazole que l'on fait réagir dans de l'éthanol absolu avec du sodium.

3. Matériau explosif comprenant la 4,6-di-(3-amino-5-nitro-1,2,4-triazole)-5-nitropyrimidine selon la revendication 1 ou 2.

## Claims

1. 4,6-Di-(3-amino-5-nitro-1,2,4-triazole)-5-nitropyrimiridine obtained by reacting the 3-amino-5-nitro-1,2,4-triazole sodium salt with 4,6-dichloro-5-nitropyrimidine, characterized by a melting point of 310°C (with decomposition), a density measured with the gas pycnometer of 1.85 and a specific surface of 2.14m²/g.

2. 4,6-Di-(3-amino-5-nitro-1,2,4-triazole)-5-nitropyrimidine according to claim 1, characterized in that the 3-amino-5-nitro-1,2,4-triazole sodium salt is obtained by acetylating the 3-amino-1,2,4-triazole in order to form the 3-acetamido-1,2,4-triazole derivative and then nitrating the thus obtained 3-acetoamido-1,2,4-triazole in order to form the 3-amino-5-nitro-1,2,4-triazole, which is reacted in absolute ethanol with sodium.

3. Explosive material incorporating 4,6-Di-(3-amino-5-nitro-1,2,4-triazole)-5-nitropyrimidine according to claim 1 or 2.

## Patentansprüche

1. 4,6-Di-(3-amino-5-nitro-1,2,4-triazol)-5-nitropyrimidin, erhalten durch Reaktion des Natriumsalzes des 3-Amino-5-nitro-1,2,4-triazols mit dem 4,6-Dichloro-5-nitropyrimidin, gekennzeichnet durch einen Schmelzpunkt von 310°C (unter Zersetzung), durch eine mit dem Gaspyknometer gemessene Dichte von 1,85 und durch eine spezifische Oberfläche von 2,14 m²/g.

2. 4,6-Di-(3-amino-5-nitro-1,2,4-triazol)-5-nitropyrimidin nach Anspruch 1, dadurch gekennzeichnet, daß das Natriumsalz des 3-Amino-5-nitro-1,2,4-triazols erhalten wird, indem man das 3-Amino-1,2,4-triazol einer Acetylierung unterwirft, um das Derivat 3-Acetamido-1,2,4-triazol zu bilden, und anschließend eine Nitrierung des so erhaltenen 3-Acetamido-1,2,4-triazols durchführt, um das 3-Amino-5-nitro-1,2,4-triazol zu bilden, das man in absolutem Ethanol mit Natrium reagieren läßt.

3. Explosivstoff, enthaltend 4,6-Di-(3-amino-5-nitro-1,2,4-triazol)-5-nitropyrimidin nach Anspruch 1 oder 2.
